# FASCICULE DE BREVET EUROPEEN

(11) **EP 3 081 257 B1**
(45) Date de publication et mention de la délivrance du brevet: **25.10.2017**
(21) Numéro de dépôt: 16164525.4
(22) Date de dépôt: 08.04.2016
(51) Int. Cl.: A61N 1/362, A61B 5/00, A61B 5/04, A61N 1/39, A61N 1/365

(54) **DISPOSITIF MÉDICAL IMPLANTABLE ACTIF DE STIMULATION CARDIAQUE COMPRENANT DES MOYENS DE DÉTECTION D'UN PHÉNOMÈNE DE REMODELAGE OU REMODELAGE INVERSE DU PATIENT**
AKTIVE IMPLANTIERBARE MEDIZINISCHE VORRICHTUNG ZUR HERZSTIMULATION, DIE DETEKTIONSMITTEL FÜR REMODELLIERUNGSERSCHEINUNGEN ODER INVERSEN REMODELLIERUNGSERSCHEINUNGEN DES PATIENTEN UMFASST
ACTIVE IMPLANTABLE MEDICAL DEVICE FOR CARDIAC STIMULATION COMPRISING MEANS FOR DETECTING A REMODELLING OR REVERSE REMODELLING PHENOMENON OF THE PATIENT

(30) Priorité: 17.04.2015 FR 1553421
(43) Date de publication de la demande: 19.10.2016
(73) Titulaire: Sorin CRM SAS, 92140 Clamart Cedex (FR)
(72) Inventeur: EUZEN, Marie-Anne, 91570 Bièvres (FR); MILPIED, Paola, 75014 Paris (FR)
(74) Mandataire: Grünecker Patent- und Rechtsanwälte PartG mbB

(56) Documents cités:
- US-A1- 2007 191 901
- US-A1- 2010 249 626
- US-A1- 2011 118 803
- US-A1- 2011 118 804
- US-A1- 2011 230 776
- US-A1- 2014 172 037

## Description

L'invention concerne les "dispositifs médicaux implantables actifs" tels que définis par la Directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

La stimulation antibradycardique implique la surveillance des potentiels électriques de dépolarisation du myocarde, et la délivrance contrôlée d'impulsions à l'oreillette et/ou au ventricule. Dans le cas d'une resynchronisation cardiaque, la stimulation doit être appliquée conjointement aux deux ventricules.

L'invention sera décrite plus précisément dans le cadre d'un implant permettant de délivrer une thérapie de resynchronisation, dite "CRT" (*Cardiac Resynchronization Therapy*) ou "BVP" (*Bi-Ventricular Pacing*), consistant à surveiller en continu le rythme cardiaque et délivrer si nécessaire au coeur des impulsions électriques permettant de stimuler de façon conjointe et permanente les ventricules gauche et droit afin de resynchroniser ces derniers.

Ce cas particulier n'est toutefois pas limitatif de l'invention qui, par un certain nombre de ses aspects, est applicable également à des dispositifs de type "simple chambre" où la détection/stimulation ne concernent que le ventricule droit, ou "double chambre" où la détection/stimulation ne concernent que le ventricule droit et l'oreillette droite.

Un stimulateur CRT est divulgué par exemple dans le EP 1 108 446 A1 (ELA Médical), qui décrit un dispositif permettant d'appliquer entre les instants respectifs de stimulation des ventricules gauche et droit un délai dit "délai interventriculaire" (DVV ou VVD) variable, ajusté de manière à resynchroniser la contraction des ventricules avec optimisation fine de l'état hémodynamique du patient. Le DVV pourra être nul, positif (le ventricule gauche étant stimulé après le ventricule droit), ou négatif (le ventricule droit étant stimulé après le ventricule gauche).

Ce dispositif CRT inclut en outre un mode de fonctionnement en stimulateur "double chambre" classique, où le dispositif surveille l'activité ventriculaire après un évènement auriculaire spontané (onde P) ou stimulé (impulsion A) et déclenche une stimulation du ventricule droit (impulsion V) si aucune activité spontanée ventriculaire (onde R) n'a été détectée à l'issue d'un délai dit "délai atrioventriculaire" ou "délai auriculo-ventriculaire" (DAV ou AVD).

L'invention concerne plus particulièrement le suivi sur le long terme de l'état cardiaque du patient et de la manière dont évolue cet état, notamment pour diagnostiquer chez celui-ci l'apparition du phénomène dénommé "remodelage cardiaque", qui peut être défini comme l'ensemble des modifications du coeur engendrées en réponse à une pathologie, et qui est généralement associé à un plus mauvais pronostic.

En effet, les modifications cliniques pouvant être asymptomatiques, il est courant que le patient adapte inconsciemment son activité à son état clinique : les premières crises d'insuffisance cardiaque apparaissant à l'effort, ceci conduit le patient à réduire son activité pour éviter la survenue de telles crises. Ensuite, les symptômes n'apparaissent plus car le patient a modifié son comportement pour les éviter, mais la pathologie continue à progresser.

Le remodelage se manifeste à la longue par une augmentation du volume du ventricule gauche, avec dégradation de la fraction d'éjection et du régime des pressions intraventriculaires du fait de la baisse de contractilité et/ou de pressions trop élevées en aval, et *in fine* par une diminution du débit cardiaque entrainant de graves conséquences sur l'organisme lorsque progresse l'insuffisance cardiaque. Et ce n'est que lorsque cette insuffisance cardiaque gênera le patient même au repos qu'il ira consulter ou, dans les cas extrêmes, devra être hospitalisé en urgence.

En résumé, du fait de l'auto-adaptation l'absence de symptômes ressentis introduit un retard important entre le début des modifications cliniques et le diagnostic de ces modifications, souvent trop tardif.

En stimulant de façon contrôlée les deux ventricules, la thérapie CRT permet d'optimiser le cycle contraction/relaxation avec un bénéfice direct facilitant le travail du coeur, ce qui peut permettre de stabiliser le phénomène de remodelage et même de le contrer ("remodelage inverse"), avec pour le patient un meilleur pronostic.

Le but principal de l'invention est de proposer un moyen de diagnostic incorporé à un stimulateur (CRT ou autre) qui permette d'assurer un suivi régulier de l'état du patient, par exemple quotidiennement, en particulier pour surveiller l'évolution d'un remodelage cardiaque (délétère) ou d'un remodelage inverse (bénéfique). Cette surveillance devra être suffisamment précoce pour pouvoir prendre au plus vite les mesures appropriées - par exemple une modification du paramétrage de la thérapie, ou la substitution à cette thérapie d'une autre thérapie si elle n'est pas efficace -, évitant ainsi le déclenchement imprévu d'une crise à brève ou moyenne échéance. Et en cas d'aggravation soudaine détectée, il sera possible d'alerter le médecin sans délai, par exemple par télétransmission d'un message à un site distant de télésurveillance.

La technique de référence pour évaluer sur le long terme l'évolution de l'état cardiaque du patient, et donc pour déterminer l'efficacité d'une thérapie et ajuster éventuellement les paramètres de stimulation, est l'échocardiographie avec mesure du volume ventriculaire et estimation des délais caractéristiques de la systole, en particulier du temps d'ouverture de la valve aortique. Cette procédure doit toutefois être mise en oeuvre en milieu hospitalier et par un personnel qualifié, elle est longue et coûteuse et ne peut pas être appliquée aussi souvent que cela serait utile ou nécessaire sans interférer avec la vie quotidienne du patient.

Des techniques d'analyse automatique à partir du dispositif implanté ont également été proposées, par exemple par le US 2011/0152660 A1, où la dégradation et l'amélioration du patient sont évaluées à partir de l'évolution du délai de conduction interventriculaire, considéré seul ou en combinaison avec l'évaluation de l'activité du patient et/ou la présence d'un oedème pulmonaire. Cette méthode est cependant très sensible à la position des sondes, ainsi qu'à la manière dont le coeur se remodèle, de sorte que dans certains cas l'analyse peut ne pas apporter l'information recherchée, notamment si aucun changement significatif du délai de conduction n'est observé selon l'axe de mesure - alors que selon un autre axe une variation beaucoup plus importante aurait pu être observée.

Le US 2007/0239037 A1 décrit une autre technique, basée sur la mesure (par échographie) du temps de conduction interventriculaire avant l'implantation du dispositif, et qui a pour objet de prédire la réponse d'un patient à une thérapie CRT : plus grand est le temps de conduction interventriculaire initial, plus la probabilité d'avoir un remodelage important sera élevée. Il ne s'agit toutefois pas d'un outil de diagnostic permettant d'évaluer sur le long terme l'évolution d'un état du patient, postérieurement à l'implantation du dispositif.

Le US 2007/0043394 A1 décrit une technique de diagnostic de la décompensation cardiaque par détection d'une variation de l'impédance intracardiaque, à fréquence cardiaque constante. L'évolution de ce paramètre sur le long terme permet de déduire si le coeur se remodèle ou non. La mesure de l'impédance intracardiaque n'est toutefois pas un paramètre stable sur le long terme (plusieurs semaines, voire plusieurs mois), ce qui en rend difficile l'exploitation à des fins diagnostiques avec un degré de certitude suffisant.

Le US 2009/0270747 A1 (US 7 996 070 B2) opère par analyse de l'électrocardiogramme (ECG) recueilli par un implant subcutané. Les modifications de la morphologie des signaux sont détectées et évaluées, en distinguant celles liées à l'état cardiaque du patient de celles liées aux changements de position de ce dernier. Mais ces enseignements ne sont pas transposables à un implant cardiaque recueillant des signaux endocavitaires d'électrogramme (EGM), qui se présentent avec une morphologie tout à fait différente.

Le but de l'invention est de proposer un dispositif de type stimulateur cardiaque implantable pourvu de moyens de diagnostic du remodelage cardiaque qui puissent pallier les inconvénients précités, en permettant en particulier :
- d'éviter des examens longs et coûteux par échographie cardiaque, qui ne fournissent qu'un diagnostic tardif, souvent plusieurs mois après l'implantation ;
- d'évaluer de façon précoce et robuste le remodelage du patient afin de réajuster en conséquence les paramètres de stimulation ;
- de mettre en place des alertes précoces en cas de risques de décompensation aggravée ou d'ischémie, pour prévenir les hospitalisations et modifier en temps utiles les thérapies et traitements ; et
- de façon générale, de s'assurer de la bonne délivrance et de l'efficacité de la thérapie appliquée.

Un autre but de l'invention est, en complément ou en variante d'un diagnostic d'un remodelage ou remodelage inverse se manifestant par des variations lentes, de pouvoir détecter des phénomènes à apparition brusque tels qu'ischémie ou déplacement de sonde, susceptibles de mettre gravement en danger le patient, ceci de manière à générer sans délai une alerte correspondante.

Plus précisément, l'invention propose un dispositif comprenant, de manière en elle-même connue :
- des moyens de stimulation contrôlée du coeur ;
- des moyens de détection de signaux de dépolarisation ventriculaire et/ou auriculaire ; et
- des moyens d'analyse morphologique, aptes à comparer entre eux des signaux de dépolarisation recueillis sur une même voie à des instants distincts.
En outre, les moyens d'analyse morphologique comprennent :
- des moyens aptes à inhiber les moyens de stimulation contrôlée ;
- des moyens aptes à recueillir concurremment sur des voies respectives distinctes, au cours d'un cycle cardiaque en rythme spontané, au moins deux signaux EGM d'électrogramme endocavitaire et en dériver au moins deux composantes temporelles distinctes respectives ; et
- des moyens aptes à combiner les au moins deux composantes temporelles en au moins une caractéristique 2D paramétrique représentative dudit cycle cardiaque, à partir des variations de l'une des composantes temporelles en fonction de l'autre ; et
- des moyens d'analyse de la caractéristique 2D, aptes à dériver de la caractéristique 2D, ou d'une moyenne de caractéristiques 2D recueillies sur des cycles cardiaques successifs, au moins un paramètre descripteur intrinsèque représentatif de la caractéristique 2D ;
- des moyens d'analyse historique, aptes à évaluer périodiquement la variation au cours du temps dudit au moins un paramètre descripteur intrinsèque d'une caractéristique 2D courante par rapport à une caractéristique 2D de référence antérieure mémorisée par le dispositif ; et
- des moyens de comparaison, aptes à comparer à au moins un seuil prédéterminé la variation évaluée par les moyens d'analyse historique, et à déclencher sélectivement une alerte en fonction du résultat de la comparaison.

Selon diverses caractéristiques subsidiaires avantageuses :
- les moyens d'analyse historique sont des moyens aptes à évaluer quotidiennement ladite variation au cours du temps du au moins un paramètre descripteur intrinsèque ;
- ledit au moins un paramètre descripteur intrinsèque est un paramètre fonction du vecteur vitesse de la caractéristique 2D, considéré en une pluralité de points respectifs (Pi) de cette caractéristique ;
- ce paramètre peut notamment comprendre la norme du vecteur vitesse, avec des moyens d'analyse historique aptes à calculer un coefficient de corrélation entre les normes des vecteurs vitesse respectifs de la caractéristique 2D courante et de la caractéristique 2D de référence ;
- ce paramètre peut également comprendre l'orientation du vecteur vitesse, avec des moyens d'analyse historique aptes à calculer l'angle moyen entre les vecteurs vitesse respectifs de la caractéristique 2D courante et de la caractéristique 2D de référence ;
- les moyens de comparaison peuvent comprendre en particulier : des moyens aptes à comparer i) l'angle moyen entre vecteurs vitesse à un premier seuil et ii) le coefficient de corrélation entre normes des vecteurs vitesse à un second seuil ; et des moyens aptes à déclencher ladite alerte lorsqu'à la fois i) l'angle moyen entre vecteurs vitesse est supérieur au premier seuil ou ii) le coefficient de corrélation entre normes des vecteurs vitesse est inférieur au second seuil ;
- les moyens d'analyse morphologique sont en outre aptes, en l'absence de déclenchement de ladite alerte par les moyens de comparaison, à actualiser ledit au moins un paramètre descripteur intrinsèque de la caractéristique 2D de référence ;
- le dispositif comprend en outre des moyens de diagnostic de remodelage ou de remodelage inverse mettant en oeuvre lesdits moyens d'analyse morphologique, ladite alerte déclenchée par les moyens de comparaison étant une alerte de survenue d'un remodelage ou remodelage inverse chez le patient ;
- le dispositif comprend en outre des moyens de détection de rupture de sonde ou de diagnostic d'ischémie mettant en oeuvre lesdits moyens d'analyse morphologique, ladite alerte déclenchée par les moyens de comparaison étant une alerte de rupture de sonde ou de survenue d'ischémie chez le patient.

Dans une forme de réalisation particulière, les signaux EGM recueillis concurremment sur des voies respectives distinctes comprennent :
- un signal EGM *far-field* unipolaire recueilli entre i) une électrode proximale ou distale ou le cas échéant une électrode intermédiaire ou un bobinage de défibrillation d'une sonde ventriculaire et ii) un boitier métallique de générateur du dispositif,
   ou bien entre i) une première électrode proximale, ou électrode distale ou bobinage de défibrillation et ii) et une seconde électrode proximale, ou une électrode distale ou le cas échéant une électrode intermédiaire ou un bobinage de défibrillation, respectivement de deux sondes ventriculaires distinctes situées toutes deux dans un même ventricule,
   ou bien entre i) une première électrode proximale, ou électrode distale ou bobinage de défibrillation et ii) et une seconde électrode proximale, ou une électrode distale ou une électrode intermédiaire, respectivement d'une sonde ventriculaire droite et d'une sonde ventriculaire gauche ; et
- un signal EGM *near-field* bipolaire recueilli entre i) une électrode distale et ii) une électrode proximale d'une sonde ventriculaire,
   ou bien entre i) un bobinage de défibrillation et ii) une électrode distale ou proximale de ladite sonde ventriculaire,
   ou bien entre i) une électrode distale et ii) une électrode intermédiaire d'une sonde ventriculaire gauche
   ou bien entre i) une électrode proximale et ii) une électrode intermédiaire de ladite sonde ventriculaire gauche
   ou bien entre deux électrodes intermédiaires de ladite sonde ventriculaire gauche.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 est une vue générale montrant un dispositif CRT avec son générateur et des sondes cardiaques droite et gauche implantées dans le coeur.
La Figure 2 est un exemple de signaux EGM obtenus sur des voies respectivement ventriculaire bipolaire et ventriculaire unipolaire de l'une des sondes de la Figure 1.
La Figure 3 illustre la manière de combiner entre eux les signaux bipolaire et unipolaire recueillis dans une même cavité ventriculaire pour construire une caractéristique bidimensionnelle de type vectogramme, indépendante du temps.
La Figure 4 est un exemple de vectogramme échantillonné obtenu pour un cycle cardiaque échantillonné à 128 Hz, avec représentation des vecteurs vitesse en divers points successifs.
La Figure 5 est un schéma de principe illustrant la mise en oeuvre de l'invention.
La Figure 6 est un diagramme présentant la séquence d'étapes exécutées lors de l'élaboration préalable des références.
La Figure 7 est un diagramme présentant la séquence d'étapes exécutées lors du suivi du patient, pour déterminer l'évolution de son état, notamment l'apparition d'un remodelage ou remodelage inverse.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu comprenant des moyens d'acquisition d'un signal fourni par des sondes endocavitaires et/ou par un ou plusieurs capteurs implantés.

L'invention peut notamment être appliquée aux dispositifs implantables tels que ceux des familles *Reply, Paradym, Intensia, Paradym RF* et *Platinium,* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé de l'invention est mis en oeuvre par des moyens principalement logiciels, grâce à des algorithmes appropriés exécutés automatiquement et de façon récurrente par un microcontrôleur ou un processeur numérique de signal. Pour la clarté de l'exposé, les divers traitements appliqués sont décomposés et schématisés par un certain nombre de blocs fonctionnels distincts, mais cette représentation n'a toutefois qu'un caractère illustratif, ces circuits comprenant des éléments communs et correspondant en pratique à une pluralité de fonctions globalement exécutées par un même logiciel.

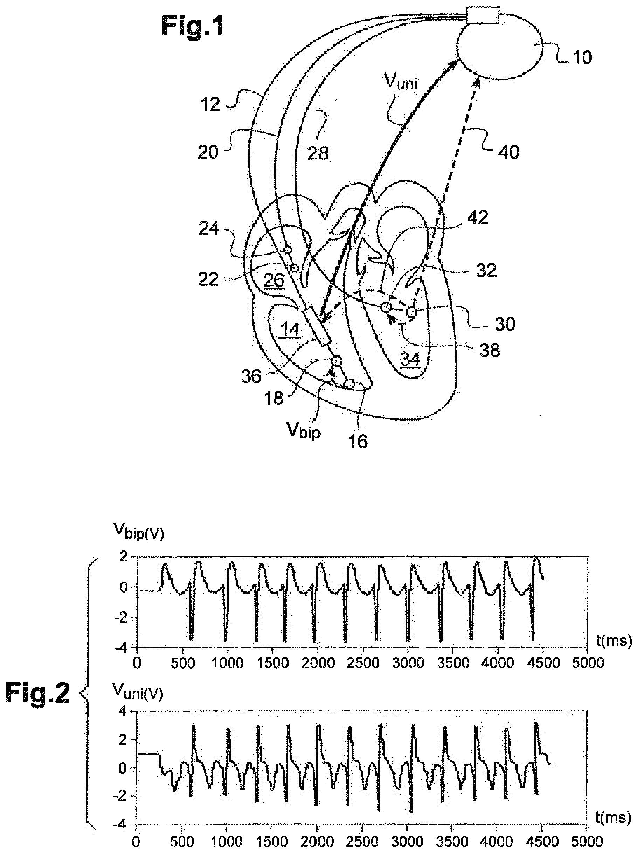

La Figure 1 illustre une configuration typique de stimulation CRT dans laquelle un générateur d'impulsions 10 est associé à une première sonde 12 implantée dans le ventricule droit 14. La tête de cette sonde comporte deux électrodes, à savoir une électrode distale (*tip*) 16 et une électrode proximale (*ring*) 18. Une deuxième sonde 20 est pourvue d'électrodes auriculaires de détection distale 22 et proximale 24 situées au niveau de l'oreillette droite 26 pour la détection des signaux dans cette cavité et l'application éventuelle d'une stimulation auriculaire.

Pour permettre la stimulation biventriculaire, notamment afin de rétablir la synchronisation entre les deux ventricules, le dispositif est pourvu d'une troisième sonde 28, par exemple une sonde disposée dans le réseau coronaire, comportant une ou plusieurs électrodes 30, 32 disposées au voisinage du ventricule gauche 34. Outre les électrodes distale et proximale 30, 32 illustrées, la sonde gauche peut également comporter une ou plusieurs électrodes intermédiaires située(s) dans une position médiane entre les électrodes 30 et 32. Il est ainsi possible d'assurer la stimulation concomitante, ou avec un léger décalage temporel contrôlé (délai interventriculaire DW), des deux ventricules droit et gauche pour rétablir la synchronisation entre ces deux cavités et améliorer l'hémodynamique générale du patient (on ajoutera que dans le cas d'une sonde gauche multi-électrodes on peut également appliquer une stimulation multisite à gauche, pour traiter un trouble du synchronisme intra-ventriculaire).

La sonde ventriculaire droite 12 peut également être pourvue d'un bobinage (*coil*) ventriculaire 36 formant électrode de défibrillation et permettant aussi de recueillir un signal endocavitaire (ce bobinage pouvant également remplacer l'électrode proximale *ring* 18).

En ce qui concerne spécifiquement la stimulation du ventricule gauche, il est possible d'utiliser une configuration bipolaire (entre les deux électrodes 30 et 32 de la sonde 28) ou unipolaire (entre l'une des électrodes 30 ou 32 et le boitier *can*) du générateur 10. Les deux "vecteurs de stimulation" correspondants sont référencés 38 et 40 sur la Figure 1. Ces mêmes vecteurs peuvent être utilisés également pour le recueil d'un signal de dépolarisation ventriculaire gauche. On notera que dans le cas d'une sonde multipolaire il existe un grand nombre de vecteurs bipolaires et unipolaires possibles, définis à partir de chacune des électrodes (on peut également utiliser simultanément plusieurs vecteurs gauches, dans l'hypothèse évoquée plus haut d'une stimulation multisite à gauche).

On va maintenant exposer, dans ce contexte, les aspects spécifiques de l'invention.

Le but de l'invention est, comme indiqué en introduction, de surveiller l'évolution sur le long terme de la morphologie des signaux cardiaques recueillis en rythme spontané du patient (c'est-à-dire en l'absence de toute stimulation appliquée par le dispositif), par exemple par une évaluation quotidienne, pour détecter :
- des variations lentes de cette morphologie, qui révèleraient notamment l'apparition d'un remodelage (avec dilatation des ventricules, délétère pour le patient) ou d'un remodelage inverse (avec diminution du volume ventriculaire, bénéfique pour le patient), et/ou
- des variations brusques, causés par des phénomènes à apparition soudaine, tels qu'ischémie ou déplacement de sonde, susceptibles de mettre gravement en danger le patient, afin de générer sans délai une alerte.

Plus précisément, pour pallier les inconvénients des techniques connues qui ont été rappelées en introduction, l'invention propose de combiner deux signaux d'électrogramme endocavitaire (EGM) recueillis en rythme spontané du patient, en particulier des signaux issus de la même cavité ventriculaire, par exemple du ventricule droit.

Pour que la condition de rythme spontané soit respectée, l'analyse des signaux est effectuée pendant un intervalle de temps où aucune stimulation n'est délivrée (dans le cas d'une stimulation à la demande), ou bien pendant une brève période d'inhibition forcée de la stimulation par le dispositif.

Les EGMs recueillis à cet effet dans le ventricule droit peuvent comprendre par exemple (voir Figure 1) :
- une composante ventriculaire droite *Vbip,* dérivée d'un signal EGM *near-field* bipolaire recueilli entre l'électrode distale 16 et l'électrode proximale 18 de la sonde ventriculaire droite 12, et
- une autre composante ventriculaire droite *Vuni,* dérivée d'un signal EGM *far-field* unipolaire recueilli entre le bobinage de défibrillation 36 de la sonde ventriculaire droite 12 et le boitier métallique du générateur 10.

La Figure 2 illustre un exemple de tracés d'EGMs *Vbip* et *Vuni* observés respectivement sur la voie bipolaire ventriculaire et la voie unipolaire ventriculaire de la configuration de la Figure 1.

D'autres configurations peuvent être utilisées, à partir de signaux de type *far-field* (par exemple entre l'une des électrodes 16 ou 18 et le boitier 10) et de type *near-field* (entre deux électrodes de la même sonde ventriculaire).

Cette configuration (signaux issus des cavités droites) n'est toutefois pas limitative. Dans certains cas, par exemple pour un patient souffrant d'un bloc de branche gauche (LBBB, *Left Bundle Branch Block*) sans problème de conduction à droite, l'effet d'un remodelage ne sera pas apparent sur les signaux recueillis au niveau des cavités droites. Dans un tel cas, il conviendra d'analyser des composantes dérivées par exemple d'un signal bipolaire ventriculaire gauche et d'un signal interventriculaire, ou encore d'un signal bipolaire gauche et d'un signal unipolaire gauche. La composante bipolaire serait alors recueillie par exemple entre les électrodes 30 et 32 de la sonde ventriculaire gauche 28 (comme référencé en 38), et la composante unipolaire entre l'électrode *tip* 30 (ou l'électrode *ring* 32) et le boitier *can* du générateur 10, comme référencé en 40. En variante, la composante unipolaire peut être également recueillie entre l'électrode *tip* 30 (ou l'électrode *ring* 32) de la sonde ventriculaire gauche 28 et le bobinage *coil* 36 de la sonde ventriculaire droite 12, comme référencé en 42. On constate que sur le long terme, si l'on effectue par exemple des comparaisons au moment de l'implantation, trois mois après l'implantation et un an après l'implantation), la forme des signaux évolue au cours du temps, mais que dans certains cas, ou chez certains patients, le signal unipolaire *Vuni* n'apporte pas vraiment d'information sur cette évolution dans le temps, tandis que le signal bipolaire *Vbip* contient des informations beaucoup plus significatives. Inversement, dans d'autres cas, l'évolution est très tardive sur le signal bipolaire *Vbip* et c'est la voie unipolaire *Vuni* (entre le bobinage coil 36 et le boitier can 10 du générateur) qui peut donner l'information recherchée, de façon beaucoup plus précoce et discriminante.

Pour pallier cet inconvénient, l'invention propose de combiner ces deux composantes bipolaire et unipolaire en une caractéristique unique contenant, de façon plus globale, toute l'information disponible afin de pouvoir procéder à une évaluation complète et robuste de l'état du patient au fil du temps.

Cette combinaison des deux signaux bipolaire et unipolaire est réalisée sous forme d'une "boucle cardiaque" ou "vectogramme" (VGM), qui est la représentation dans un espace à deux dimensions de l'un des deux signaux EGM (en ordonnée) par rapport à l'autre (en abscisse). Chaque cycle cardiaque est alors représenté par un vectogramme dans le plan {*Vbip,Vuni*} ainsi défini, vectogramme dont la géométrie (forme de la courbe) fait donc abstraction de la dimension temporelle - qui n'intervient que comme un paramètre décrivant la manière dont la courbe est parcourue.

On soulignera que ce "vectogramme" (VGM), qui est obtenu à partir de signaux d'électrogramme (EGM) issu de sondes intracardiaques, ne doit pas être confondu avec le "vectocardiogramme" (VCG) qui est, lui, obtenu à partir de signaux d'électrocardiogramme (ECG) issu d'électrodes externes placées sur le thorax du patient.

La construction d'un VGM et son analyse pour quantifier des données cardiaques sont décrites par exemple dans Milpied et al., "Arrhythmia Discrimination in Implantable Cardioverter Defibrillators using Support Vector Machines applied to a new Representation of Electrograms," IEEE Transactions on Biomedical Engineering, June 2011, 58(6):1797-1803. L'analyse d'un VGM à des fins différentes de celles de l'invention a été déjà proposée notamment par le EP 2 105 843 (ELA Médical) pour la discrimination entre tachycardies ventriculaires et supraventriculaires, par le EP 2 324 885 A1 (Sorin CRM) pour l'invalidation d'un test de capture en cas de fusion, c'est-à-dire de stimulation déclenchée de façon concomitante à une dépolarisation spontanée, par le EP 2 368 493 A1 (Sorin CRM) pour la discrimination d'artefacts de bruit pour la validation ou l'invalidation de cycles cardiaques à analyser, par le EP 2 742 971 A1 (Sorin CRM) pour déterminer la présence ou l'absence d'une onde évoquée induite par la stimulation d'une cavité, ou encore par le EP 2 742 973 A1 (Sorin CRM) pour détecter la présence éventuelle d'un phénomène de stimulation anodique.

Mais dans tous ces cas, il s'agit d'analyser un cycle cardiaque isolé, pour révéler une caractéristique propre à ce cycle (type d'arythmie, présence ou non d'une capture, artefact, etc.). Il ne s'agit jamais, comme dans la présente invention, d'effectuer une analyse historique visant à déterminer l'apparition de variations plus ou moins rapides, sur le long terme, de telle ou telle caractéristique du VGM, notamment à des fins de diagnostic d'un état évolutif du patient.

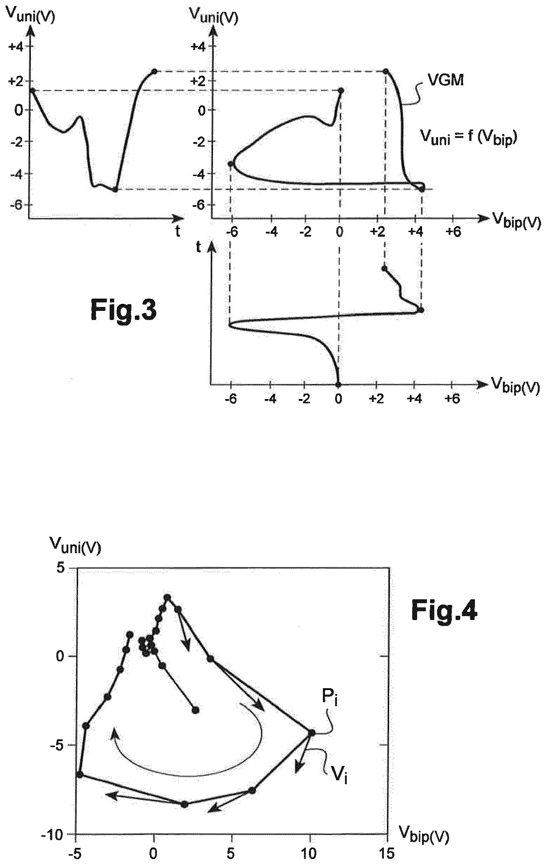

Concrètement, comme illustré Figure 3, les signaux EGM *Vuni*(*t*) et *Vbip*(*t*) recueillis sont échantillonnés, et les échantillons successifs des deux composantes sont mémorisés puis combinés entre eux pour produire une courbe paramétrique (la caractéristique VGM) du type VGM = (*Vbip*(*t*)*, Vuni*(*t*)) ou {x = *Vbip*(t)*,* y = *Vuni*(t)}*.*

Cette courbe est une courbe paramétrée par le temps, tracée à partir des variations de l'une des composantes temporelles (*Vuni*) en fonction de l'autre (*Vbip*)*.* Elle constitue un vectogramme (VGM) représentatif du cycle cardiaque à analyser, et sera également désignée "caractéristique 2D paramétrique". Elle présente graphiquement la forme d'une boucle, le temps n'apparaissant plus que dans la manière dont la boucle est parcourue sur la durée du cycle.

On notera incidemment que l'analyse 'bidimensionnelle" ou "en deux dimensions" (2D) évoquée ici ne doit pas être entendue de manière en elle-même limitative. L'invention peut en effet s'appliquer aussi bien à une analyse dans un espace multidimensionnel d'ordre supérieur (3D ou plus), par extrapolation des enseignements de la présente description à une situation où des signaux EGM provenant d'une même cavité sont recueillis simultanément sur trois voies ou plus.

En pratique, comme illustré Figure 4, l'échantillonnage produit un VGM en forme de polygone ouvert où chaque sommet correspond à un point d'échantillonnage de la mesure du signal *Vuni* et *Vbip* de l'EGM. Dans l'exemple de la Figure 4, l'échantillonnage est opéré à une fréquence de 128 Hz, ce qui donne environ 11 points de mesure pour un intervalle de temps de 80 ms, qui sont autant de valeurs qui peuvent être mises en mémoire pour être analysées.

On a également représenté sur la Figure 4 l'allure du vecteur vitesse Vᵢ en divers points successifs Pi du VGM pour une fréquence d'échantillonnage de 128 Hz. En un point donné, la vitesse est une donnée vectorielle (la vitesse étant définie par son orientation et sa norme), et le vecteur vitesse peut être calculé en chaque point du VGM à partir d'un filtre discret qui approxime les dérivées premières *Vbip*(t)*l*dt et *Vuni*(t)*l*dt qui, pour une caractéristique échantillonnée, peuvent être calculées à partir du point précédent et du point suivant sur la courbe.

La caractéristique VGM recueillie est mémorisée sous forme d'une série de paramètres descripteurs basés sur les vecteurs vitesse en chaque point de la courbe et comprenant i) la norme du vecteur vitesse et ii) l'orientation du vecteur vitesse, c'est-à-dire l'angle qu'il fait par rapport à l'axe des abscisses du VGM.

L'invention propose d'opérer une comparaison morphologique entre le VGM courant (mémorisé sous forme des valeurs des normes et des angles des vecteurs vitesse aux différents points d'échantillonnage) avec un VGM de référence (mémorisé sous forme de descripteurs homologues).

La comparaison entre ces deux VGMs consiste à quantifier leurs ressemblances à partir :
- du coefficient de corrélation C entre les normes des vecteurs vitesse respectifs du VGM courant et du VGM de référence, et
- de la valeur moyenne θ de l'angle que font entre eux les vecteurs vitesse respectifs du VGM courant et du VGM de référence.

On peut considérer que les courbes se ressemblent, si C (qui reflète la corrélation entre les normes des vecteurs vitesse) est suffisamment grand et θ (qui reflète les écarts angulaires d'orientation) est suffisamment petit. Un simple seuil peut alors être défini pour chaque descripteur (SEUIL1 pour l'angle et SEUIL2 pour le coefficient de corrélation), afin de conclure sur la ressemblance des courbes :
- si θ est inférieur à SEUIL1 et C supérieur à SEUIL2, on considèrera que les courbes sont similaires,
- si θ est supérieur à SEUIL1 ou C est inférieur à SEUIL2, on considèrera que les courbes sont différentes.

Un remodelage ou un remodelage inverse ne devraient pas modifier brusquement la morphologie des EGMs recueillis en rythme spontané. Si l'on trouve des courbes différentes, on peut donc penser qu'une anomalie est survenue.

L'invention propose de détecter d'éventuels changements de cette morphologie des EGMs, que ces changements soient brusques ou qu'ils soient progressifs.

Dans le cas d'un changement à la fois brusque et important de morphologie, on peut considérer que le patient est en danger, ce changement révélant un phénomène grave tel qu'un déplacement de sonde, une ischémie, etc. Il est alors souhaitable de déclencher une alerte.

Des changements progressifs, mais peu importants, de morphologie peuvent se présenter notamment après les premiers mois suivant l'implantation, et sont dus par exemple à la maturation des sondes. Aucune action particulière n'est à entreprendre dans ce cas.

En revanche, des changements dus à un remodelage ou à un remodelage inverse seront également progressifs, mais plus importants. Si l'on veut mettre en évidence ces phénomènes particuliers, il suffira de choisir des seuils appropriés, par exemple déterminés au préalable à partir d'une base d'apprentissage.

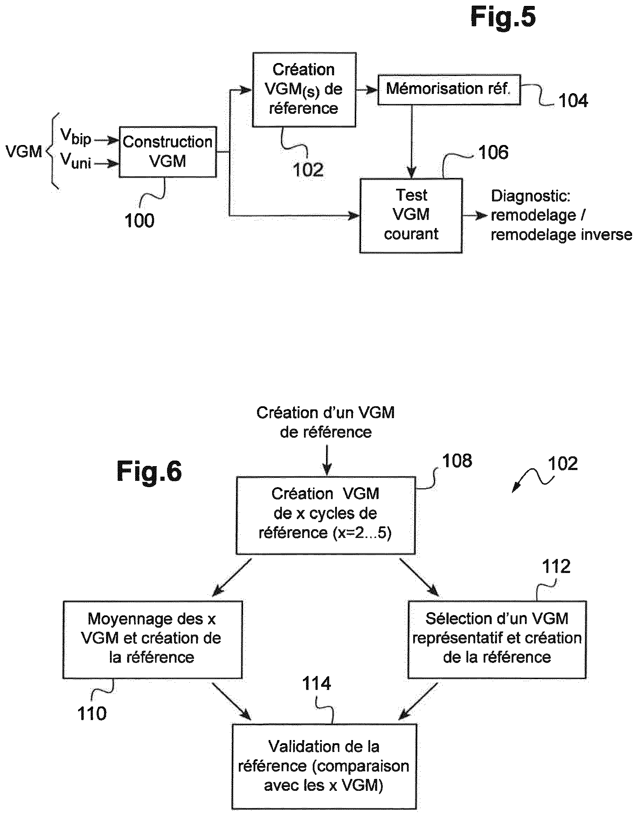

La Figure 5 illustre de façon très générale la manière dont est mis en oeuvre le dispositif de l'invention.

Le VGM courant est construit périodiquement, par exemple une fois par jour ou une fois par semaine, dans des conditions stables (rythme sinusal lent, de préférence la nuit), pour être ensuite mémorisé et comparé à un VGM de référence créé à l'initialisation de la thérapie, et éventuellement mis à jour en cas d'alerte ou de décompensation, ou de façon manuelle par le praticien lors d'une visite de suivi par exemple.

À partir des EGMs bipolaire et unipolaire recueillis, un VGM est construit (bloc 100). Dans un premier temps, un (ou plusieurs) VGM(s) de référence est (sont) constitué(s) (bloc 102, détaillé Figure 8) et conservé(s) en mémoire (bloc 104). De la même façon, périodiquement, par exemple quotidiennement, un VGM courant est construit et comparé à la référence mémorisée (bloc 106) pour en déduire un indicateur de l'évolution sur le long terme de l'état du patient (remodelage, remodelage inverse ou état non évolutif) et/ou l'apparition soudaine d'un phénomène grave tel que rupture de sonde ou ischémie.

La Figure 6 illustre la création d'un VGM de référence (bloc 102 de la Figure 5).

Cette référence est créée à partir de plusieurs cycles cardiaques représentatifs (absence d'artefact, d'extrasystole, de fusion, etc.), par exemple x = 2 à 5 cycles cardiaques, pour chacun desquels le VGM est construit et le vecteur de paramètres descripteurs est calculé (bloc 108). Ces données sont ensuite moyennées (bloc 110) ou, en variante, l'un des cycles cardiaques le plus représentatif est sélectionné pour constituer la référence (bloc 112). À partir du VGM moyenné ou sélectionné, la référence est validée (bloc 114), en la comparant à chacun des cycles recueillis à l'étape 108, de manière à vérifier que l'écart entre cette référence et chacun des autres cycles reste dans des limites acceptables, ou en s'assurant qu'il reste dans ces limites pour un nombre suffisant, prédéterminé, de ces autres cycles.

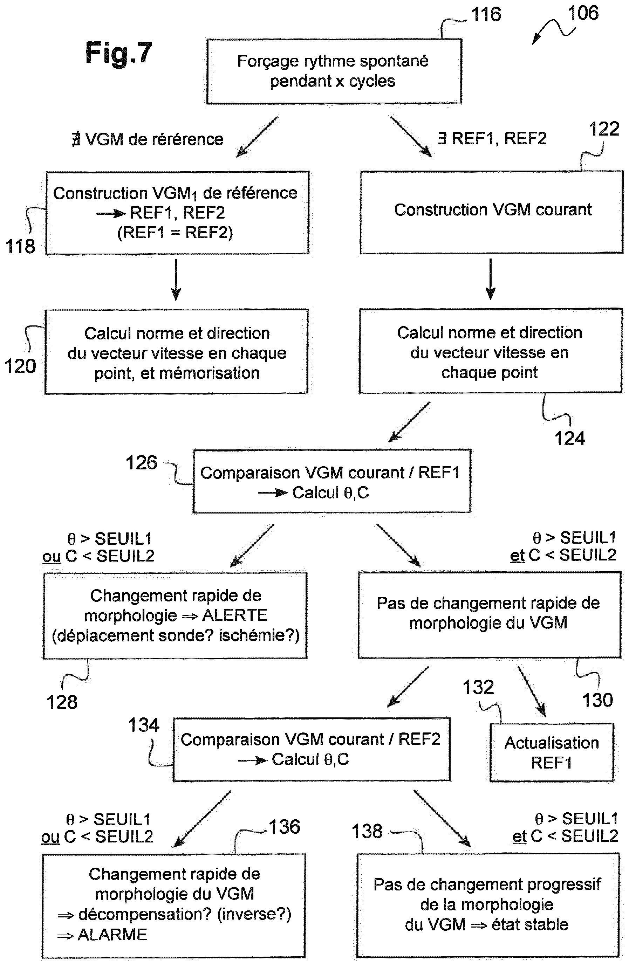

La Figure 7 illustre la séquence des différentes opérations du test du VGM courant (bloc 106 de la Figure 5) effectué à intervalles réguliers, par exemple chaque jour.

La première étape (bloc 116) est l'inhibition de toute stimulation par le dispositif, de manière à laisser s'exprimer un rythme spontané pendant un nombre prédéterminé x de cycles.

Si aucun VGM de référence n'est disponible à ce stade, il convient de créer un ou plusieurs VGMs de référence correspondant à l'état du patient. Dans l'exemple illustré, on créera deux VGMs de référence REF1, REF2 :
- le VGM de référence REF1 sera destiné à mettre en évidence les changements brutaux de morphologie, résultant par exemple d'un déplacement de sonde ou de l'apparition d'une ischémie, tandis que
- le VGM de référence REF2 sera destiné à la mise en évidence des changements progressifs de morphologie liés à l'apparition éventuelle d'un remodelage ou remodelage inverse ; ce VGM sera déterminé une fois pour toutes et ne sera pas modifié automatiquement ensuite.

Les VGMs de référence REF1 et REF2 sont construits de la manière expliquée plus haut en référence à la Figure 6, avec initialement REF1 = REF2 (bloc 118). Dans le cas d'un échantillonnage à 128 Hz, le VGM est par exemple construit sur un intervalle d'environ 80 ms centré sur le pic de dépolarisation du ventricule droit, à partir de 11 échantillons successifs. Les normes et les directions des vecteurs vitesse du VGM sont ensuite déterminées et mémorisées (bloc 120).

Si, à l'issue de l'étape 116 les références REF1 et REF2 sont disponibles en mémoire, alors le dispositif construit le VGM courant (bloc 122) et détermine la norme et la direction du vecteur vitesse en chaque point de ce VGM (bloc 124), de la même manière qu'aux blocs 118 et 120 correspondant aux VGMs de référence.

L'étape suivante (bloc 126) consiste à calculer les descripteurs C (coefficients de corrélation entre les normes des vecteurs vitesse) et θ (angle moyen entre les vecteurs vitesse) représentatifs de la comparaison entre le VGM courant et le VGM de référence REF1.

Si la condition θ > SEUIL1 ou C < SEUIL2 est vérifiée (bloc 128) ceci signifie qu'il y a un changement brusque de morphologie est intervenu, et justifie le déclenchement d'une alerte, par exemple la télétransmission d'un message à un site distant afin d'alerter un médecin sur un phénomène tel que déplacement de sonde ou apparition d'une ischémie.

Si la condition précitée n'est pas vérifiée, c'est-à-dire si θ < SEUIL1 et C > SEUIL2 (bloc 130) on considère qu'il n'y a pas eu de changement rapide de la morphologie. Le VGM de référence REF1 est alors actualisé en le remplaçant par la valeur du VGM courant (bloc 132), et le dispositif cherche alors à mettre en évidence un changement progressif de morphologie qui pourrait révéler un remodelage ou remodelage inverse.

À cet effet, les descripteurs C et θ sont calculés (bloc 134) de la même manière qu'au bloc 126, mais cette fois-ci en comparant le VGM courant au VGM de référence REF2.

Si la condition θ > SEUIL1 ou C < SEUIL2 est vérifiée (bloc 136) on considère qu'il y a eu une modification significative de la morphologie, pouvant laisser supposer un remodelage. Une alerte est éventuellement délivrée, correspondant à ce résultat.

Afin de décider si une alerte est nécessaire, d'autres paramètres peuvent être pris en compte, comme la largeur du complexe QRS de l'EGM courant (cette largeur étant calculée comme étant le temps écoulé entre le début et la fin de la dépolarisation détectée), ou encore un ou plusieurs paramètres dérivés d'un signal d'accélération endocardiaque (EA), ou également le temps de conduction interventriculaire. En effet, un raccourcissement important de la largeur du complexe QRS est probablement dû à un remodelage inverse - qui ne justifie pas d'alerte -, de même que l'augmentation de l'amplitude du pic d'accélération endocardiaque ou une diminution du temps de conduction interventriculaire.

Dans tous les cas contraires, c'est-à-dire si θ < SEUIL1 et C > SEUIL2 (bloc 138), on considère qu'il n'y a eu aucun changement, ni brusque ni progressif de la morphologie du VGM, et donc que l'état du patient est stable, sans aggravation ni amélioration.

## Revendications

1. Un dispositif médical implantable actif (10) de stimulation, défibrillation et/ou resynchronisation cardiaque, comportant :
- des moyens de stimulation contrôlée du coeur d'un patient ;
- des moyens de détection de signaux de dépolarisation ventriculaire et/ou auriculaire ; et
- des moyens d'analyse morphologique, aptes à comparer entre eux des signaux de dépolarisation recueillis sur une même voie à des instants distincts,
**caractérisé en ce que** les moyens d'analyse morphologique comprennent :
- des moyens (116) aptes à inhiber les moyens de stimulation contrôlée ;
- des moyens aptes à recueillir concurremment sur des voies respectives distinctes, au cours d'un cycle cardiaque en rythme spontané, au moins deux signaux EGM d'électrogramme endocavitaire et en dériver au moins deux composantes temporelles (*Vbip, Vuni*) distinctes respectives ; et
- des moyens (122) aptes à combiner les au moins deux composantes temporelles (*Vbip, Vuni*) en au moins une caractéristique 2D paramétrique (VGM) représentative dudit cycle cardiaque, à partir des variations de l'une des composantes temporelles en fonction de l'autre ; et
- des moyens (124) d'analyse de la caractéristique 2D, aptes à dériver de la caractéristique 2D, ou d'une moyenne de caractéristiques 2D recueillies sur des cycles cardiaques successifs, au moins un paramètre descripteur intrinsèque représentatif de la caractéristique 2D ;
- des moyens (126, 134) d'analyse historique, aptes à évaluer périodiquement la variation au cours du temps dudit au moins un paramètre descripteur intrinsèque d'une caractéristique 2D courante par rapport à une caractéristique 2D de référence antérieure mémorisée par le dispositif ; et
- des moyens (126, 134) de comparaison, aptes à comparer à au moins un seuil prédéterminé (SEUIL1, SEUIL2) la variation évaluée par les moyens d'analyse historique, et à déclencher sélectivement une alerte d'aggravation de l'état cardiaque du patient en fonction du résultat de la comparaison.

2. Le dispositif de la revendication 1, dans lequel les moyens (126, 134) d'analyse historique sont des moyens aptes à évaluer quotidiennement ladite variation au cours du temps du au moins un paramètre descripteur intrinsèque.

3. Le dispositif de la revendication 1, dans lequel ledit au moins un paramètre descripteur intrinsèque est un paramètre fonction du vecteur vitesse (Vᵢ) de la caractéristique 2D, considéré en une pluralité de points respectifs (Pi) de cette caractéristique.

4. Le dispositif de la revendication 3, dans lequel ledit paramètre fonction du vecteur vitesse comprend la norme du vecteur vitesse (Vᵢ).

5. Le dispositif de la revendication 4, dans lequel lesdits moyens (126, 134) d'analyse historique comprennent des moyens aptes à calculer un coefficient de corrélation (C) entre les normes des vecteurs vitesse respectifs de la caractéristique 2D courante et de la caractéristique 2D de référence.

6. Le dispositif de la revendication 3, dans lequel ledit paramètre fonction du vecteur vitesse comprend l'orientation du vecteur vitesse (Vᵢ).

7. Le dispositif de la revendication 6, dans lequel lesdits moyens (126, 134) d'analyse historique comprennent des moyens aptes à calculer l'angle moyen (θ) entre les vecteurs vitesse respectifs de la caractéristique 2D courante et de la caractéristique 2D de référence.

8. Le dispositif des revendications 5 et 7 prises en combinaison, dans lequel les moyens de comparaison (126, 134) comprennent :
- des moyens aptes à comparer i) l'angle moyen (θ) entre vecteurs vitesse à un premier seuil (SEUIL1) et ii) le coefficient de corrélation (C) entre normes des vecteurs vitesse à un second seuil (SEUIL2) ; et
- des moyens aptes à déclencher ladite alerte lorsqu'à la fois i) l'angle moyen (θ) entre vecteurs vitesse est supérieur au premier seuil (SEUIL1) ou ii) le coefficient de corrélation (C) entre normes des vecteurs vitesse est inférieur au second seuil (SEUIL2).

9. Le dispositif de la revendication 1, dans lequel les moyens d'analyse morphologique comprennent en outre :
- des moyens (132) aptes, en l'absence de déclenchement de ladite alerte par les moyens de comparaison, à actualiser ledit au moins un paramètre descripteur intrinsèque de la caractéristique 2D de référence.

10. Le dispositif de la revendication 1, comprenant en outre des moyens (136) de diagnostic de remodelage ou de remodelage inverse mettant en oeuvre lesdits moyens d'analyse morphologique, ladite alerte déclenchée par les moyens de comparaison étant une alerte de survenue d'un remodelage ou remodelage inverse chez le patient.

11. Le dispositif de la revendication 1, comprenant en outre des moyens (128) de détection de rupture de sonde ou de diagnostic d'ischémie mettant en oeuvre lesdits moyens d'analyse morphologique, ladite alerte déclenchée par les moyens de comparaison étant une alerte de rupture de sonde ou de survenue d'ischémie chez le patient.

12. Le dispositif de la revendication 1, dans lequel lesdits au moins deux signaux EGM recueillis concurremment sur des voies respectives distinctes comprennent :
- un signal EGM *far-field* unipolaire (*Vuni*) recueilli entre i) une électrode proximale (18) ou distale (16) ou le cas échéant une électrode intermédiaire ou un bobinage de défibrillation (36) d'une sonde ventriculaire et ii) un boitier métallique de générateur (10) du dispositif,
ou bien entre i) une première électrode proximale, ou électrode distale ou bobinage de défibrillation et ii) et une seconde électrode proximale, ou une électrode distale ou le cas échéant une électrode intermédiaire ou un bobinage de défibrillation, respectivement de deux sondes ventriculaires distinctes situées toutes deux dans un même ventricule,
ou bien entre i) une première électrode proximale, ou électrode distale ou bobinage de défibrillation et ii) et une seconde électrode proximale, ou une électrode distale ou une électrode intermédiaire, respectivement d'une sonde ventriculaire droite et d'une sonde ventriculaire gauche, et
- un signal EGM *near-field* bipolaire (*Vbip*) recueilli entre i) une électrode distale (16) et ii) une électrode proximale (18) d'une sonde ventriculaire (12),
ou bien entre i) un bobinage de défibrillation (36) et ii) une électrode distale (16) ou proximale (18) de ladite sonde ventriculaire (12),
ou bien entre i) une électrode distale et ii) une électrode intermédiaire d'une sonde ventriculaire gauche (28)
ou bien entre i) une électrode proximale et ii) une électrode intermédiaire de ladite sonde ventriculaire gauche (28)
ou bien entre deux électrodes intermédiaires de ladite sonde ventriculaire gauche (28).

## Patentansprüche

1. Aktive implantierbare medizinische Vorrichtung (10) zur Herz-Stimulation, - Defibrillation und/oder Resynchronisation, aufweisend:
- Mittel zur kontrollierten Stimulation des Herzens eines Patienten;
- Mittel zur Erfassung von ventrikulären und/oder atrialen Depolarisationssignalen; und
- Mittel zur morphologischen Analyse, die dazu geeignet sind, auf einem gleichen Weg, zu verschiedenen Zeitpunkten, aufgefangene Depolarisationssignale zu vergleichen,
**dadurch gekennzeichnet, dass** die Mittel zur morphologischen Analyse Folgendes umfassen:
- Mittel (116), die dazu geeignet sind, die Mittel zur kontrollierten Stimulation zu hemmen;
- Mittel, die dazu geeignet sind, während eines Herzzyklus mit Eigenrhythmus, gleichzeitig auf jeweils verschiedenen Wegen, mindestens zwei EGM-Signale eines endokavitären Elektrogramms aufzufangen und daraus mindestens zwei jeweils verschiedene zeitliche Komponenten (*Vbip, Vuni*) abzuleiten; und
- Mittel (122), die dazu geeignet sind, die mindestens zwei zeitlichen Komponenten (*Vbip, Vuni*) in mindestens eine parametrische 2D-Charakteristik (VGM), die den Herzzyklus repräsentiert, anhand von Variationen einer der zeitlichen Komponenten als Funktion der jeweils anderen, zu kombinieren; und
- Mittel (124) zur Analyse der 2D-Charakteristik, die dazu geeignet sind, aus der 2D-Charakteristik oder aus einem Durchschnitt von über aufeinanderfolgenden Herzzyklen aufgefangenen 2D-Charakteristiken mindestens einen intrinsischen deskriptiven Parameter, der die 2D-Charakteristik repräsentiert, abzuleiten;
- Mittel (126, 134) zur historischen Analyse, die dazu geeignet sind, periodisch die zeitliche Variation des mindestens einen intrinsischen deskriptiven Parameters einer aktuellen 2D-Charakteristik im Vergleich zu einer früheren in der Vorrichtung gespeicherten 2D-Referenzcharakteristik zu bemessen; und
- Vergleichsmittel (126, 134), die dazu geeignet sind, die durch die Mittel zur historischen Analyse bemessene Variation mit mindestens einer vorbestimmten Schwelle (SEUIL1, SEUIL2) zu vergleichen, und in Abhängigkeit des Ergebnisses des Vergleichs selektiv einen über eine Verschlimmerung des Herzzustandes des Patienten informierenden Alarm auszulösen.

2. Vorrichtung nach Anspruch 1, wobei die Mittel (126, 134) zur historischen Analyse Mittel sind, die dazu geeignet sind, die zeitliche Variation des mindestens einen intrinsischen deskriptiven Parameters täglich zu beurteilen.

3. Vorrichtung nach Anspruch 1, wobei der mindestens eine intrinsische deskriptive Parameter ein Parameter ist, der von dem Geschwindigkeitsvektor (Vᵢ) der 2D-Charakteristik abhängt, der an mehreren jeweiligen Punkten (Pᵢ) dieser Charakteristik betrachtet wird.

4. Vorrichtung nach Anspruch 3, wobei der von dem Geschwindigkeitsvektor abhängige Parameter, die Norm des Geschwindigkeitsvektors (Vᵢ) umfasst.

5. Vorrichtung nach Anspruch 4, wobei die Mittel (126, 134) zur historischen Analyse Mittel umfassen, die dazu geeignet sind, einen Korrelationskoeffizienten (C) zwischen den Normen der jeweiligen Geschwindigkeitsvektoren der aktuellen 2D-Charakteristik und der 2D-Referenzcharakteristik zu berechnen.

6. Vorrichtung nach Anspruch 3, wobei der von dem Geschwindigkeitsvektor abhängige Parameter, die Ausrichtung des Geschwindigkeitsvektors (Vᵢ) umfasst.

7. Vorrichtung nach Anspruch 6, wobei die Mittel (126, 134) zur historischen Analyse Mittel umfassen, die dazu geeignet sind, einen mittleren Winkel (θ) zwischen den jeweiligen Geschwindigkeitsvektoren der aktuellen 2D-Charakteristik und der 2D-Referenzcharakteristik zu berechnen.

8. Vorrichtung nach den Ansprüchen 5 und 7 in Kombination miteinander, wobei die Vergleichsmittel (126, 124) Folgendes umfassen:
- Mittel, die dazu geeignet sind, i) den mittleren Winkel (θ) zwischen Geschwindigkeitsvektoren mit einer ersten Schwelle (SEUIL1) und ii) den Korrelationskoeffizienten (C) zwischen Normen der Geschwindigkeitsvektoren mit einer zweiten Schwelle (SEUIL2) zu vergleichen; und
- Mittel, die dazu geeignet sind, den Alarm sowohl dann auszulösen, wenn i) der mittlere Winkel (θ) zwischen den Geschwindigkeitsvektoren grösser als die erste Schwelle (SEUIL1) ist oder ii) der Korrelationskoeffizient (C) zwischen den Normen der Geschwindigkeitsvektoren kleiner als die zweite Schwelle (SEUIL2) ist.

9. Vorrichtung nach Anspruch 1, wobei die Mittel zur morphologischen Analyse des Weiteren Folgendes umfassen:
- Mittel (132), die dazu geeignet sind, in Abwesenheit einer Auslösung des Alarms durch die Vergleichsmittel, den mindestens einen intrinsischen deskriptiven Parameter der 2D-Referenzcharakteristik zu aktualisieren.

10. Vorrichtung nach Anspruch 1, weiter umfassend Mittel (136) zur Diagnostizierung einer Remodellierung oder inversen Remodellierung, welche die Mittel zur morphologischen Analyse einsetzen, wobei der durch die Vergleichsmittel ausgelöste Alarm ein Alarm über das Eintreten einer Remodellierung oder inversen Remodellierung beim Patienten ist.

11. Vorrichtung nach Anspruch 1, weiter umfassend Mittel (128) zur Erfassung eines Sonden-Bruchs oder zur Diagnostizierung einer Ischämie, welche die Mittel zur morphologischen Analyse einsetzen, wobei der durch die Vergleichsmittel ausgelöste Alarm ein Alarm über den Bruch einer Sonde oder das Auftreten einer Ischämie beim Patienten ist.

12. Vorrichtung nach Anspruch 1, wobei die mindestens zwei gleichzeitig auf jeweils verschiedenen Wegen aufgefangenen EGM-Signale Folgendes umfassen:
- ein unipolares *Far-Field-*EGM-Signal (*Vuni*)*,* das zwischen i) einer proximalen (18) oder distalen (16) oder gegebenenfalls einer dazwischenliegenden Elektrode oder einer Defibrillierwicklung (36) einer ventrikularen Sonde und ii) einem metallischen Generatorgehäuse (10) der Vorrichtung,
oder zwischen i) einer ersten proximalen Elektrode oder distalen Elektrode oder Defibrillierwicklung und ii) einer zweiten proximalen Elektrode oder einer distalen Elektrode oder gegebenenfalls einer dazwischenliegenden Elektrode oder einer Defibrillierwicklung, von jeweils zwei verschiedenen in einem selben Ventrikel befindlichen ventrikularen Sonden,
oder zwischen i) einer ersten proximalen Elektrode oder distalen Elektrode oder Defibrillierwicklung und ii) einer zweiten proximalen Elektrode oder einer distalen Elektrode oder einer dazwischenliegenden Elektrode, jeweils einer rechten ventrikularen Sonde und einer linken ventrikularen Sonde aufgefangen wird, und
- ein bipolares *Near*-*Field*-EGM-Signal (*Vbip*), das zwischen i) einer distalen Elektrode (16) und ii) einer proximalen Elektrode (18) einer ventrikularen Sonde (12),
oder zwischen i) einer Defibrillierwicklung (36) und ii) einer distalen (16) oder proximalen (18) Elektrode der ventrikularen Sonde (12),
oder zwischen i) einer distalen Elektrode und ii) einer dazwischenliegenden Elektrode einer linken ventrikularen Sonde (28),
oder zwischen i) einer proximalen Elektrode und ii) einer dazwischenliegenden Elektrode der linken ventrikularen Sonde (28)
oder zwischen zwei dazwischenliegenden Elektroden der linken ventrikularen Sonde (28) aufgefangen wird.

## Claims

1. An active implantable medical device (10) for stimulation, defibrillation and/or cardiac resynchronization, comprising:
- means for controlled stimulation of a patient's heart;
- means for detecting ventricular and/or atrial depolarization signals; and
- means for a morphological analysis capable of comparing depolarization signals collected on a same channel at distinct moments,
**characterized in that** the means for morphological analysis comprise:
- means (116) that are capable of inhibiting the means for controlled stimulation;
- means that are capable of concurrently collecting, on respective distinct channels, during a spontaneous rhythm cardiac cycle, at least two EGM signals of an endocavitary electrogram and deriving at least two respective distinct temporal components (*Vbip, Vuni*) from them ; and
- means (122) that are capable of combining the at least two temporal components (*Vbip, Vuni*) into at least one parametric 2D characteristic (VGM) representative of said cardiac cycle, based on the variations of one of the temporal components as a function of the other temporal component; and
- means (124) for analyzing the 2D characteristic, capable of deriving, from the 2D characteristic or from an average of 2D characteristics collected over successive cardiac cycles, at least one intrinsic descriptor parameter of the 2D characteristic;
- means (126, 134) for historical analysis, capable of periodically evaluating the variation over time of said at least one intrinsic descriptor parameter of a current 2D characteristic with respect to a previous reference 2D characteristic stored by the device;
- means (126, 134) for comparison, capable of comparing the variation evaluated by the means for historical analysis to at least one predetermined threshold (SEUIL1, SEUIL2) and selectively triggering an alert signaling a worsening of the patient's cardiac condition as a function of the comparison result.

2. The device according to claim 1, wherein the means (126, 134) for historical analysis are means capable of evaluating, on a daily basis, the variation over time of the at least one intrinsic descriptor parameter.

3. The device according to claim 1, wherein the at least one intrinsic descriptor parameter is a function of the velocity vector (Vᵢ) of the 2D characteristic, considered in a plurality of respective points (Pᵢ) of the characteristic.

4. The device according to claim 3, wherein the parameter that is a function of the velocity vector comprises the norm of the velocity vector (Vᵢ).

5. The device according to claim 4, wherein the means (126, 134) for historical analysis comprise means capable of calculating a correlation coefficient (C) between the norms of the respective velocity vectors of the current 2D characteristic and the reference 2D characteristic.

6. The device according to claim 3, wherein the parameter that is a function of the velocity vector comprises the orientation of the velocity vector (Vᵢ).

7. The device according to claim 6, wherein the means (126, 134) for historical analysis comprise means capable of calculating the average angle (θ) between the respective velocity vectors of the current 2D characteristic and the reference 2D characteristic.

8. The device according to claims 5 and 7 combined, wherein the means for comparison (126, 134) comprise:
- means capable of comparing i) the average angle (θ) between velocity vectors to a first threshold (SEUIL1) and ii) the correlation coefficient (C) between norms of the velocity vectors with a second threshold (SEUIL2); and
- means capable of triggering the alert both when i) the average angle (θ) between velocity vectors is higher than the first threshold (SEUIL1) or ii) the correlation coefficient (C) between norms of the velocity vectors is lower than the second threshold (SEUIL2).

9. The device according to claim 1, wherein the means for morphological analysis further comprise:
- means (132) capable of updating the at least one intrinsic descriptor parameter of the reference 2D characteristic when the alert is not triggered by the comparison.

10. The device according to claim 1, further comprising means (136) for performing diagnostics of remodeling or of reverse remodeling using the means for morphological analysis, the alert triggered by the means of comparison being an alert of an occurrence of remodeling or of an occurrence of reverse remodeling in the patient.

11. The device according to claim 1, further comprising means (128) for detecting breakage of a probe or diagnosing ischemia using the means for morphological analysis, the alert triggered by the means of comparison being an alert of breakage of a probe or the occurrence of ischemia in the patient.

12. The device according to claim 1, wherein the at least two EGM signals collected concurrently on separate respective channels comprise:
- a unipolar EGM far-field signal (*Vuni*) collected between i) a proximal (18) or distal (16) electrode or, where appropriate, an intermediary electrode or a defibrillation coil (36) of a ventricular probe and ii) a metal casing of a generator (10) of the device,
or between i) a first proximal electrode or a distal electrode or a defibrillation coil and ii) a second proximal electrode, or a distal electrode or, where appropriate, an intermediary electrode or a defibrillation coil, respectively of two distinct ventricular probes both located in the same ventricle,
or between i) a first proximal electrode, or distal electrode or defibrillation coil and ii) a second proximal electrode or a distal electrode or an intermediary electrode, respectively of a right hand ventricular probe and a left hand ventricular probe, and
- a near-field EGM bipolar signal (*Vbip*) collected between i) a distal electrode (16) and ii) a proximal electrode (18) of the ventricular probe (12),
or between i) a defibrillation coil (36) and ii) a distal (16) or proximal (18) of the ventricular probe (12),
or between i) a distal electrode and ii) an intermediary electrode of a left hand ventricular probe (28)
or between i) a proximal electrode and ii) an intermediary electrode of the left hand ventricular probe (28)
or between two intermediary electrodes of the left hand ventricular probe (28).
